# EUROPEAN PATENT APPLICATION

(11) **EP 4 011 447 A1**
(43) Date of publication of application: **15.06.2022**
(21) Application number: 20849417.9
(22) Date of filing: 06.08.2020
(51) Int. Cl.: A61P 17/14, A61Q 7/00, C07K 14/78, C12N 5/077, C12N 5/10, A61K 8/98, C12N 15/12, A61K 35/36

(54) **METHOD FOR CULTURING DERMAL PAPILLA CELLS**

(30) Priority: 07.08.2019 JP 2019145099
(71) Applicant: KAO CORPORATION, Tokyo 103-8210 (JP); OSAKA UNIVERSITY, Suita-shi Osaka 565-0871 (JP)
(72) Inventor: YAMASHITA, Shiho, Haga-gun, Tochigi 321-3497 (JP); NAKAGIRI, Yoriko, Haga-gun, Tochigi 321-3497 (JP); SEKIGUCHI, Kiyotoshi, Suita-shi, Osaka 565-0871 (JP); SHIMONO, Chisei, Suita-shi, Osaka 565-0871 (JP)
(74) Representative: Vossius & Partner Patentanwälte Rechtsanwälte mbB
(86) International application number: PCT/JP2020/030218
(87) International publication number: WO 2021/025122

(57) **Abstract**

Provided is a method for efficiently culturing dermal papilla cells while maintaining an ability to induce hair follicles. A method for culturing dermal papilla cells in the presence of at least one selected from the group consisting of EMILIN and a fragment thereof.

## Description

### Field of the Invention

The present invention relates to a method for culturing dermal papilla cells.

### Background of the Invention

Dermal papilla cells are mesenchymal cells that exist at the base of hair follicles and play a role of inducing sending a differentiation signal to follicular epithelial stem cells to thereby induce differentiation.

In other words, dermal papilla cells are indispensable cells together with follicular epithelial stem cells for hair follicle regeneration, and the collection of dermal papilla cells from a small number of hair follicles and the mass culturing of the cells would contribute to hair regeneration therapy and studies on the hair regeneration.

However, the ability of dermal papilla cells to induce hair follicles will be lost as the number of culture passages increases. For this reason, a technique of culturing dermal papilla cells while maintaining the ability to induce hair follicles has been needed and several attempts have been reported, including culturing dermal papilla cells in the presence of BMP-2/BMP-4 products and WNT products (Patent Literature 1), culturing on feeder cells (Patent Literature 2), culturing in a spheroid form (Non Patent Literature 1) and the like.

(Patent Literature 1) WO2010/021245
(Patent Literature 2) JP-B-5164439

(Non Patent Literature 1) ACS Appl Master Interfaces, 2016, 8: 5906-5916

### Summary of the Invention

The present invention relates to the following invention.
1) A method for culturing dermal papilla cells, comprising culturing dermal papilla cells in the presence of at least one selected from the group consisting of EMILIN and a fragment thereof.
2) A method for maintaining or facilitating an ability of dermal papilla cells to induce hair follicles, comprising culturing dermal papilla cells in the presence of at least one selected from the group consisting of EMILIN and a fragment thereof.
3) An agent for maintaining or facilitating an ability of dermal papilla cells to induce hair follicles, comprising as an active ingredient at least one selected from the group consisting of EMILIN and a fragment thereof.
4) Use of at least one selected from the group consisting of EMILIN and a fragment thereof for producing an agent for maintaining or facilitating an ability of dermal papilla cells to induce hair follicles.
5) Use of at least one selected from the group consisting of EMILIN and a fragment thereof for maintaining or facilitating an ability of dermal papilla cells to induce hair follicles.
6) At least one selected from the group consisting of EMILIN and a fragment thereof for use in maintaining or facilitating an ability of dermal papilla cells to induce hair follicles.

### Brief Description of the Drawings

[Figure 1] Figure 1 is micrographic photographs of cultured dermal papilla cells stained for the alkaline phosphatase activity.
[Figure 2] Figure 2 is graphs showing changes in various gene expressions of cultured dermal papilla cells.
[Figure 3] Figure 3 is micrographic photographs showing the proliferation of cultured pluripotent stem cell-derived dermal papilla cells.
[Figure 4] Figure 4 is graphs showing changes in various gene expressions of cultured pluripotent stem cell-derived dermal papilla cells.

### Detailed Description of the Invention

An object of the present invention is to provide a method for efficiently culturing dermal papilla cells while maintaining the ability to induce hair follicles.

The present inventors conducted various studies on culture conditions of dermal papilla cells and found that dermal papilla cells can be proliferated while maintaining the ability to induce hair follicles when cultured under the condition in which EMILIN or a fragment thereof is present.

The present invention enables an easy proliferation of dermal papilla cells without decreasing the ability to induce hair follicles. Due to this, *in vitro* experiments using cultured dermal papilla cells having the ability to induce hair follicles are easier, and hair regeneration is also easier for isolating and culturing dermal papilla cells collected from own hair of a patient and transferring them back to the patient.

In the present invention, the "dermal papilla cells" are mesenchymal cells located at the bottom of hair follicles and play a role of sending an activation signal to follicular epithelial stem cells for the self-renewal of hair follicles. Dermal papilla cells are locally present in the hair papilla and are clearly distinguished from mesenchymal stem cells locally present in the bone marrow, fat, joint synovial membrane, tooth pulp, and the like.

Dermal papilla cells used in the present invention can be those derived from skins of the mammals such as human, mouse, rat, rabbit, guinea pig, goat, pig, and cow, but human skin-derived dermal papilla cells are preferable. The skin can be selected from not only skin pertaining to hair on the head but also skins pertaining to hair on the body as long as the normal physiological functions of the hair papilla are retained, but the scalp is preferable. Usable human scalp can be collected by, for example, a surgery, and dermal papilla cells are collected from anagen hair follicles isolated under a microscope.

Dermal papilla cells to be cultured can be prepared from the collected skin using a known technique (for example, Tissue Eng. 2007, 13:975-82.). In other words, anagen hair follicles are isolated from the collected scalp tissues under a stereomicroscope, and the isolated dermal papilla cells are then allowed to stand on a culture dish and cultured for several days in a DMEM medium containing 10% bovine serum and FGF-2 under the conditions of 37°C, 5% CO₂, and a humidity of 100% in a CO₂ incubator, thereby isolating dermal papilla cells.

Additionally, dermal papilla cells to be used in the present invention can also be pluripotent stem cell-derived dermal papilla cells prepared by inducing differentiation of pluripotent stem cells such as embryonic stem cells (ES cells), embryonic carcinoma cells (EC cells), embryonic germ cells (EG cells), and iPS cells. For the pluripotent stem cells, human-derived pluripotent stem cells are preferable, and human iPS cells are more preferable. For the pluripotent stem cell-derived dermal papilla cells, skin-derived pluripotent precursor cells (SKPs) and the like prepared by inducing differentiation of pluripotent stem cells by a known method (for example, JP-B-6304818) can be used as the dermal papilla cells.

In the present invention, the culture of dermal papilla cells is carried out in the presence of at least one selected from the group consisting of EMILIN and a fragment thereof.

The "EMILIN" refers to the elastin microfibril interface-located protein, which is the glycoprotein making up the extracellular matrix and having a molecular weight of 115 kDa. To date, EMILIN-1, EMILIN-2, and EMILIN-3 have been found and form the EMILIN protein family.

Of these, EMILIN-1, with the functions thereof having been revealed, is reported to be present at the interface of the elastin and fine fibers in the skin and involved in the elasticity of the skin by adjusting the elastin deposition (The Journal of Japanese Society of Periodontology 2004 46: 175-184).

The EMILIN has gC₁q domain, which is an integrin binding site, at the C-terminal side and the cysteine-rich EMI at the N-terminal side and are involved in the multimerization. The EMILIN forms a homotrimer to perform functions thereof.

The EMILIN of the present invention is not particularly limited in the origin, and EMILIN of various creatures, with mammal-derived EMILIN being preferable. Examples of the mammal include human, mouse, rat, cow, and pig but are not limited thereto. Of these, human-derived EMILIN is particularly preferably used. Additionally, a recombinant can also be acceptable.

Information on the genes and amino acid sequences of human EMILIN can be obtained from known databases (GenBank and the like), and registrations are made under NP_008977.1 for human EMILIN-1, NP_114437.2 for human EMILIN-2, and NP_443078.1 for human EMILIN-3. Of these, human EMILIN-1 is preferable, and the amino acid sequence thereof is set forth in SEQ ID NO: 1.

Examples of the EMILIN fragment include a partial peptide of EMILIN such as a C-terminal fragment. Partial peptides of EMILIN-1 are preferable. The gC₁q domain is an integrin binding site located at a 120 to 160 amino acid region from the C-terminal side (J Biol Chem. 2003, 278: 6160-6167.). Thus, the EMILIN fragment of the present invention can be deemed preferably a partial peptide of EMILIN which have the integrin binding site. The presence of the integrin binding activity of an EMILIN fragment can be confirmed by the ELISA method and the like.

In the present invention, examples of the preferable EMILIN fragment specifically include a C-terminal fragment of EMILIN-1, a fragment of EMILIN-1 containing the gC₁q domain, more preferably a C-terminal fragment of EMILIN-1 consisting of 120 to 200 amino acids, in detail, a C-terminal fragment of EMILIN-1 containing the gC₁q domain, and in more detail, a C-terminal fragment of EMILIN-1 containing the gC₁q domain and consisting of 120 to 160 amino acids.

The EMILIN or an EMILIN fragment of the present invention may be a protein or a peptide consisting of an amino acid sequence in which one or several amino acids are deleted, substituted, or added in the protein or peptide, and have the integrin binding activity. Examples of such protein or peptide include a protein or a peptide consisting of an amino acid sequence in which one or several amino acids are deleted, substituted, or added in the part other than the gC₁q domain in the protein or peptide.

The several amino acids herein can be, for example 2 to 10 amino acids, 2 to 8 amino acids, 2 to 6 amino acids, and 2 to 4 amino acids.

The EMILIN can be produced by, for example, a method of purifying cells expressing the EMILIN at a high level or as a recombinant protein. The method for producing an EMILIN fragment is not particularly limited and examples thereof include a method of digesting the full-length EMILIN with a protease, fractionating a target fragment, and purifying, and a method for producing as a recombinant protein. A recombinant EMILIN and a recombinant EMILIN fragment can be produced by suitably using a known genetic recombination technique. In other words, a recombinant EMILIN or a recombinant EMILIN fragment can be produced by obtaining DNA each encoding a full-length protein or a partial protein, inserting the DNA in an expression vector, introducing the vector into suitable host cells thereby expressing the DNA, and purifying the protein forming a trimer by a known method.

The EMILIN or a fragment thereof mentioned above can contain a sequence used for the construction, separation, or purification thereof, such as a tag sequence and a linker sequence. In other words, the EMILIN or a fragment thereof of the present invention encompass, as an embodiment, those having an amino acid such as a tag sequence added to the terminus of EMILIN or an EMILIN fragment, for example, those having a 6 x His tag sequence added to the N-terminus.

The culture conditions for dermal papilla cells of the present invention can be any conditions that allows the proliferation of dermal papilla cells and are not particularly limited, provided that the culture is carried out in the presence of at least one selected from the group consisting of EMILIN and a fragment thereof. For example, dermal papilla cells may be cultured on a culture substrate containing at least one selected from the group consisting of EMILIN and a fragment thereof, or dermal papilla cells may be cultured by adding to a culture medium at least one selected from the group consisting of EMILIN and a fragment thereof.

Examples of the culture substrate containing at least one selected from the group consisting of EMILIN and a fragment thereof include a culture substrate having a coating agent containing at least one selected from the group consisting of EMILIN and a fragment thereof (for examples, plates, meshes, and dishes), and examples thereof include a culture substrate to which at least one selected from the group consisting of EMILIN and a fragment thereof is adsorbed by coating with a coating agent containing at least one selected from the group consisting of EMILIN and a fragment thereof.

When at least one selected from the group consisting of EMILIN and a fragment thereof is added to a culture medium, at least one selected from the group consisting of EMILIN and a fragment thereof may be added to a culture medium prior to seeding dermal papilla cells, or at least one selected from the group consisting of EMILIN and a fragment thereof may be added to a culture medium together with dermal papilla cells.

The culture substrate, the coating agent, and the culture medium containing at least one selected from the group consisting of EMILIN and a fragment thereof can contain a scaffolding material or a cell adhesion molecule other than EMILIN or a fragment thereof. Examples of the scaffolding material or cell adhesion molecule other than EMILIN or a fragment thereof include extracellular matrix structural components such as collagen, elastin, laminin, proteoglycan, and glycosaminoglycan, and of these, laminin, collagen type I or collagen type IV, which are extracellular matrix structural components equivalent to the hair root, are suitably selected.

The content of the EMILIN or a fragment thereof in a culture substrate containing at least one selected from the group consisting of EMILIN and a fragment thereof may be preferably from 0.05 to 50 µg, more preferably from 0.1 to 10 µg, and more preferably from 0.2 to 3 µg per cm² of area of the culture substrate brought into contact with the culture. For example, the culture substrate is coated with a coating agent containing preferably 0.05 to 50 µg, more preferably 0.1 to 10 µg, and more preferably 0.2 to 3 µg of EMILIN or a fragment thereof per cm² of area to be coated to adsorb the EMILIN or a fragment thereof to the culture substrate.

The content of the EMILIN or a fragment thereof in a culture medium to which at least one selected from the group consisting of EMILIN and a fragment thereof is added can be adjusted to be preferably 0.1 to 100 µg, and more preferably 0.2 to 20 µg, as the final concentration per mL of the culture medium.

The culture container is not particularly limited in the material or shape as long as cells can be cultured. For example, plastic and glass can be used for the material, and wells with a bottom shape such as roundbottom or cone shape can also be used in addition to dishes.

Specifically, examples thereof include tissue culture dishes such as a cell culture surface treated dish manufactured by Corning (product number: 430165) and a tissue culture dish manufactured by IWAKI (product number: 3000-035).

The culture solution (culture medium) can be a commercial nutrient culture medium used for culturing animal cells without further modification, or can be modified by adding an additive and used. Examples of the known culture media include MEM culture medium (Minimum Essential Medium), BME culture medium (Basal Medium Eagle), IMDM culture medium (Iscove's Modified Dulbecco's Medium), D-MEM culture medium (Dulbecco's Modified Eagle's Medium), Ham's culture medium (Ham's F12), RPMI culture medium (Roswell Park Memorial Institute medium), Fischer's culture medium, dermal papilla cells proliferation culture medium, and mixed culture media thereof.

The additive may be a serum-containing culture medium, a serum-free culture medium, or a culture medium containing a serum substitute. Examples of the serum substitute include albumin, transferrin, fatty acids, collagen precursors, trace elements (for example, zinc and selenium), nutrition factors (EGF (epidermal growth factor) and bFGF (basic fibroblast growth factor)), B-27 (trademark) supplement, N2 supplement, knockout serum replacement, and 2-mercaptoethanol. Examples thereof further include, as needed, components typically used in a culture medium such as vitamins, buffers, inorganic salts, antibiotics (for example, penicillins, kanamycin, streptomycin).

The cell seeding density at the start of culture is not particularly limited and, for example, 0.15 × 10⁴ to 10 × 10⁴ cells/cm², and preferably 0.5 × 10⁴ to 2.0 × 10⁴ cells/cm², per culture container.

The culture conditions are not particularly limited, and the culture temperature is, for example, 30 to 40°C, and preferably 36°C to 38°C, and the CO₂ concentration is, for example, 3 to 10%, and preferably 4 to 6%. The oxygen concentration is, for example, 1 to 25%, and preferably 2 to 20%. The culture is preferably continued until a cell colony density reaches about 80% with respect to a culture container.

The thus cultured dermal papilla cells maintain the ability to induce hair follicles. The presence of the ability to induce hair follicles can be confirmed by evaluating the alkaline phosphatase (ALP) activity whose relevance to the ability to induce hair follicles has been reported, the presence or absence of the expression of proteins and the genes encoding such proteins for realizing the ability to induce hair follicles, or the cell morphology by microscopic observation. For example, the expression of a protein can be confirmed by a method utilizing the antigen-antibody reaction, and the gene expression can be confirmed by a method utilizing the northern blot method, the polymerase chain reaction (PCR) and the like.

Examples of the molecules, which increases the expression when the ability to induce hair follicles enhances, include alkaline phosphatase (ALP), Wingless related MMTV integration site 5A (WNT5A; involved in hair follicles development during the fetal stage), Bone morphogenetic protein 4 (BMP4; involved in hair follicles development during the fetal stage), Lymphoid enhancer binding factor 1 (LEF1; involved in the expression suppression of E-cadhelin at the time of developing hair follicles anlage), and Low-density lipoprotein receptor-related protein 4 (LRP4; dermal papilla cell marker), and Dickkopf-1 (DKK-1; expressed at a high level in alopecia patients) is reported as the molecule, which decreases the expression, when the ability to induce hair follicles enhances (literature; J Cell Sci. 2012 125: 4114-4125).

Thus, the method for culturing dermal papilla cells in the presence of at least one selected from the group consisting of EMILIN and a fragment thereof according to the present invention can maintain or facilitate the ability of dermal papilla cells to induce hair follicles. Consequently, EMILIN or a fragment thereof can be an agent for maintaining or facilitating the ability to induce hair follicles to be used by, for example, adding to a culture medium when dermal papilla cells are cultured.

In the present invention, the following embodiments are further disclosed in relation to the aspects mentioned above.
<1> A method for culturing dermal papilla cells comprising culturing dermal papilla cells in the presence of at least one selected from the group consisting of EMILIN and a fragment thereof.
<2> The method of <1>, wherein the culturing is carried out on a culture substrate comprising at least one selected from the group consisting of EMILIN and a fragment thereof, or carried out by adding to a culture medium at least one selected from the group consisting of EMILIN and a fragment thereof.
<3> The method of <2>, wherein the culturing is carried out by adding at least one selected from the group consisting of EMILIN and a fragment thereof in a culture container coated with a scaffolding material or a cell adhesion molecule other than EMILIN or a fragment thereof.
<4> The method of any of <1> to <3>, wherein the content of the EMILIN or a fragment thereof in a culture medium is adjusted to 0.1 to 100 µg, and preferably 0.2 to 20 µg, as the final concentration per mL of the culture medium.
<5> The method of <2>, wherein, the content of the EMILIN or a fragment thereof in the culture substrate comprising at least one selected from the group consisting of EMILIN and a fragment thereof is adjusted to 0.05 to 50 µg, preferably 0.1 to 10 µg, and more preferably 0.2 to 3 µg per cm² of area of the culture substrate brought into contact with the culture.
<6> The method of any of <1> to <5>, wherein the EMILIN is EMILIN-1.
<7> The method of any of <1> to <5>, wherein the fragment of the EMILIN comprises gC₁q domain.
<8> The method of <7>, wherein the fragment of the EMILIN is a C-terminal fragment of EMILIN-1.
<9> The method of <7>, wherein the fragment of the EMILIN is a C-terminal fragment of EMILIN-1 consisting of 120 to 200 amino acids.
<10> A method for maintaining or facilitating an ability of dermal papilla cells to induce hair follicles, comprising culturing dermal papilla cells in the presence of at least one selected from the group consisting of EMILIN and a fragment thereof.
<11> An agent for maintaining or facilitating an ability of dermal papilla cells to induce hair follicles, comprising as an active ingredient at least one selected from the group consisting of EMILIN and a fragment thereof.
<12> Use of at least one selected from the group consisting of EMILIN ad a fragment thereof for producing an agent for maintaining or facilitating an ability of dermal papilla cells to induce hair follicles.
<13> Use of at least one selected from the group consisting of EMILIN and a fragment thereof for maintaining or facilitating an ability of dermal papilla cells to induce hair follicles.
<14> At least one selected from the group consisting of EMILIN and a fragment thereof for use in maintaining or facilitating an ability of dermal papilla cells to induce hair follicles.
<15> In the above <11> to <14>, the EMILIN is EMILIN-1.
<16> In the above <11> to <14>, the fragment of the EMILIN comprises gC₁q domain.
<17> In the above <16>, the fragment of the EMILIN is a C-terminal fragment of EMILIN-1.
<18> In the above <16>, the fragment of the EMILIN is a C-terminal fragment of EMILIN-1 consisting of 120 to 200 amino acids.

### Examples

### Reference Example 1 Preparation of a human recombinant EMILIN-1 C-terminal fragment

As an EMILIN fragment, human recombinant EMILIN-1 C-terminal fragments (hereinafter referred to as "EM1-C") were prepared by the method shown below.

At first, PCR was carried out using the following 3 kinds of primer sets and, as a template, cDNAs obtained by reverse-transcribing Human Fetal Heart Total RNA (Clontech Laboratories, Inc.) using SuperScript^{™} III First-Strand Synthesis System (product name, Invitrogen, Corp.), and the cDNAs encoding human EMILIN-1 fragments were amplified.
(i) Human EMILIN-1 first fragment amplification primers 5'-atatatgctagccactgtggagcgccccgccatg-3' (forward, SEQ ID NO: 2) 5'-tccctgccccgcggctcctc-3' (reverse, SEQ ID NO: 3)
(ii) Human EMILIN-1 second fragment amplification primers
   5'-atgtcgtggccggctcagtgacagtg-3' (forward, SEQ ID NO:4)
   5'-cctcctgctgcagcctgttaatctcagaaatgatacggtc-3' (reverse, SEQ ID NO: 5)
(iii) Human EMILIN-1 third fragment amplification primers
   5'-gaccgtatcatttctgagattaacaggctgcagcaggagg-3' (forward, SEQ ID NO: 6)
   5'-atatataagcttctaatgatgatgatgatgatgcgcgtgttcaagctctgggtcccc -3' (reverse, SEQ ID NO: 7)

Then, PCR was carried out using the following primer set and, as a template, cDNA encoding human EMILIN-1 second fragment and third fragment, and cDNA encoding the fragment linking the second fragment and the third fragment was amplified.
(iv) Amplification primers for cDNA encoding the fragment combining the second and the third fragments
5'-atgtcgtggccggctcagtgacagtg-3' (forward, same as SEQ ID NO: 4)
5'-atatataagcttctaatgatgatgatgatgatgcgcgtgttcaagctctgggtcccc -3' (reverse, same as SEQ ID NO: 7)

The cDNA encoding human EMILIN-1 first fragment was enzymatically digested with restriction enzymes NheI and SacII, and the cDNA encoding the fragment linking the second and the third fragments was enzymatically digested with restriction enzymes SacII and HindIII.

Mammalian cell expression vector pSecTag2A (Invitrogen, Corp.) was cleaved with restriction enzymes NheI and HindIII, each of the cDNA encoding human EMILIN-1 fragments enzymatically digested as above was inserted to this site thereby to prepare the expression vector of human full-length EMILIN-1.

Then, PCR was carried out using the following primers and the expression vector of human full-length EMILIN-1 as a template, and cDNA encoding the human EMILIN-1 C-terminal fragment (Ala⁸⁴⁵-Ala⁹⁹⁵ polypeptide chain [human EMILIN-1 amino acid sequence excluding secretory signal with the N-terminal residue Ala as the first position]) was amplified.
(v) Human EMILIN-1 C-terminal fragment amplification primers
5'-ccaggttccactggtgaccatcatcatcatcatcatgaggagggacaagcacaggcc ggc-3' (forward, SEQ ID NO: 8)
5'-atatataagcttctacgcgtgttcaagctctgggtccccatagag-3' (reverse, SEQ ID NO: 9)

Additionally, PCR was carried out using the following primers and the mammalian cell expression vector pSecTag2A (Invitrogen, Corp.) as a template, and cDNA encoding the IgK secretory signal of the pSecTag2A expression vector (Met-Glu-Thr-Asp-Thr-Leu-Leu-Leu-Trp-Val-Leu-Leu-Leu-Trp-Val-Pro-Gly-Ser-Thr-Gly-Asp (SEQ ID NO: 10)) and a 6xHis tag was amplified.
(vi) IgK Secretory signal amplification primers
5'-CGGTAGGCGTGTACGGTGGG-3' (forward, SEQ ID NO: 11)
5'-atgatgatgatgatgatggtcaccagtggaacctggaaccc-3' (reverse, SEQ ID NO: 12)

PCR was carried out using the following primers and the cDNA encoding the IgK secretory signal prepared above and the cDNA encoding human EMILIN-1 C-terminal fragments as templates, and cDNA encoding human recombinant EMILIN-1 C-terminal fragment (EM1-C) (containing the IgK secretory signal and the 6xHis tag at the N-terminal side) was amplified.
(vii) EM1-C-Encoding cDNA amplification primers
5'-CGGTAGGCGTGTACGGTGGG-3' (forward, same as SEQ ID NO: 11)
5'-atatataagcttctacgcgtgttcaagctctgggtccccatagag-3' (reverse, same as SEQ ID NO: 9)

The amplified cDNA was excised with restriction enzymes NheI and HindIII, and inserted to this site of the mammalian cell expression vector pSecTag2A (Invitrogen, Corp.), thereby to prepare the expression vector of EM1-C.

The expression of EM1-C was carried out by introducing the prepared expression vector into human kidney-derived 293F cells (purchased from Invitrogen, Corp.). 600 µg of the expression vector was transfected into 600 mL of 293F cells (1.0 × 10⁶ cells/mL) using 780 µL of a transfection reagent 293 fectin (product name, Invitrogen, Corp.) and 42 mL of Opti-MEM (product name, Invitrogen, Corp.), and cultured for 48 hours, and then the culture solution was collected. The culture solution was centrifuged at 1,000 g for 15 minutes, and the supernatant thereof was further centrifuged at 15,000 x g for 30 minutes to remove cells and insoluble matters. The culture supernatant was transferred to a plastic Erlenmeyer flask (Corning), 1 mL of cOmplete His-tag Purification Resin (product name, Roche, Ltd.), a protease inhibitor Pefabloc (product name, Roche, Ltd., final concentration diluted 2,000 times), imidazole (final concentration 5 mM), and sodium azide (final concentration 0.05%) were added thereto, and the mixture was incubated with rotation at 4°C overnight to cause the adsorption of the target proteins by the batch method. The suspension was transferred to an econo column, cOmplete His-tag Purification Resin was collected, washed with 5 mM imidazole/TBS(-) (Ca and Mg *free* tris-buffered saline), and then eluted with 250 mM imidazole/TBS(-). The eluted fractions were combined and transferred to a centrifugal ultrafiltration filter (NMWL: 30 K, Millipore, Corp.), and centrifugally concentrated. The concentrated fraction was transferred to a dialysis cassette (MWCO: 20 K, Thermo Fisher Scientific), and the dialysis was carried out to PBS(-) (Ca and Mg free phosphate buffered saline). The dialyzed purified protein solution was collected, filter-sterilized using a 0.22 µm disk filter, and quantitatively determined by the BCA method. The purified EM1-C (containing 6xHis tag at the N-terminal side) was confirmed for the purity by silver staining after SDS-PAGE.

### Example 1 Culture of dermal papilla cells of normal human hair on head

### 1. Cell culture

Dermal papilla cells of normal human hair on head (TOYOBO: CA602t05a) were seeded on a collagen type I-coated dish 100 mm (IWAKI: 4020-010) and cultured using a dermal papilla cell proliferation culture medium (TOYOBO: TMTPGM-250). Using Accutase (Innovative Cell Technologies: AT104), 80% to 90% confluent dermal papilla cells were peeled, seeded on a collagen type I-coated dish 35 mm (IWAKI: 4000-010) or on a 6-well plate (Falcon: 353046) in which 2 mL of 100 nM EM1-C was added per well and allowed to stand at 37°C for 1 hour in such a way as to be 0.67 × 10⁴/cm² or 1.0 × 10⁴/cm² respectively and cultured using a dermal papilla cell proliferation culture medium in an incubator at 37°C, 5%CO₂ for 3 days.

### 2. Confirmation of the ability to induce hair follicles (Alkaline phosphatase staining)

The cultured dermal papilla cells were alkaline phosphatase stained. The alkaline phosphatase staining was carried out using Blue Alkaline Phosphatase Substrate Kit (Vector laboratories: SK-5300) in accordance with the attached protocol.

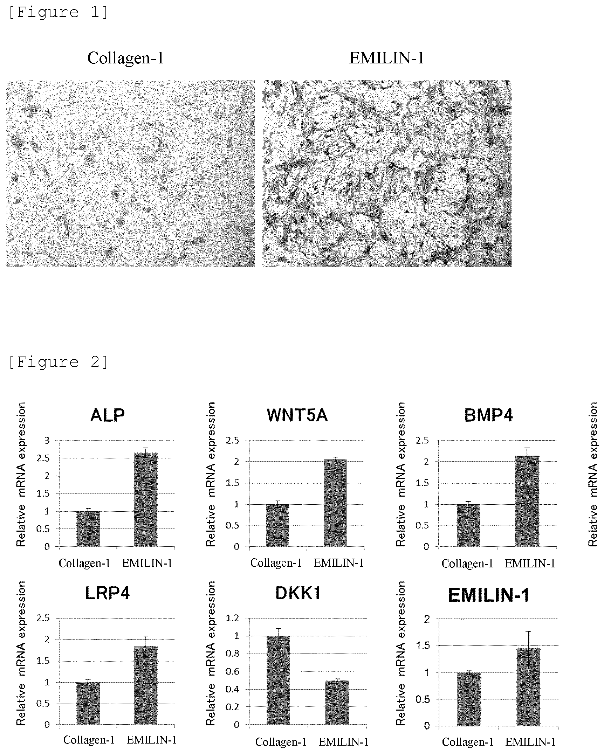

Figure 1 shows the results of alkaline phosphatase-stained cells. The dermal papilla cells cultured on the EM1-C-coated plate were more intensely stained by the alkaline phosphatase activity staining than the dermal papilla cells cultured on the collagen type I-coated plate.

From the above results, it is believed that EMILIN-1 improves the ability of dermal papilla cells to induce hair follicles.

### 3. Gene expression of dermal papilla cells

Gene expression state of the cultured dermal papilla cells was investigated by the quantitative PCR method. RNA was extracted from each of the samples using RNeasy Mini kit (QIAGEN: 74104). Each total RNA concentration was measured, and reverse transcription was carried out using a given amount of the total RNA. For the reverse transcript, High capacity RNA-to-cDNA Kit (Applied Biosystems: 4387406) was used. The gene expression by the quantitative PCR method was detected and quantified using 1 µL of the obtained cDNA sample and Taqman Probe (Applied Biosystems: 4448892) by QuantStudio Realtime PCR system (Applied Biosystems). The amplification conditions were denaturation reactions for 95 and 15 seconds in a 20-µL reaction system, 1 minute-annealing at 60°C, and an extension reaction. For each of the gene reaction levels, a value calculated by the ΔCt method was corrected by using an expression level of RPLP0, and the gene expression levels are each expressed as a relative value with the expression level in dermal papilla cells cultured on the collagen type I set to 1.0.

Figure 2 shows the results on the gene expression of dermal papilla cells investigated by the quantitative PCR method. It was confirmed that the dermal papilla cells cultured on the plate coated with EM1-C had an increased expression of Alkaline Phosphatase (ALP), which has been implicated in the ability to induce hair follicles, as compared with the dermal papilla cells cultured on the plate coated with the collagen type I. Further confirmed were increased expressions of Wingless related MMTV integration site 5A (WNT5A) and Bone morphogenetic protein 4 (BMP4) involved in hair follicle development during the fetal stage, and Lymphoid enhancer binding factor 1 (LEF1) which suppresses the E-cadhelin expression at the time of developing hair follicles anlage. It was also confirmed that low-density lipoprotein receptor-related protein 4 (LRP4), which is a dermal papilla cell marker, had an increased expression, whereas Dickkopf-1 (DKK-1), which is expressed at a high level in alopecia patients, had a decreased expression. Additionally, an increased expression of the EMILIN-1 gene was confirmed when dermal papilla cells were cultured on EM1-C.

From the above results, it is believed that EMILIN-1 has a potential to improve the ability of dermal papilla cells to induce hair follicles.

### Example 2 Culture of pluripotent stem cell-derived dermal papilla cells on EMILIN-1

### <Preparation of pluripotent stem cell-derived dermal papilla cells>

### 1. Preparation of skin-derived pluripotent precursor cells (SKPs)

### (1) Culture of iPS cells

Human-derived iPS cells (clone name: 1231A3) was used as the pluripotent stem cells. Maintenance culture of the cells was performed in accordance with the culture method recommended by the acquisition organization. In other words, using StemFit (registered trademark) (AK02N, manufactured by Ajinomoto Co., Inc.) as the human iPS cell culture medium, the cells were cultured in an incubator of 37°C, 5%CO₂ in accordance with the method described in Sci. Rep, 4, 2014, 3594; DOI:10.1038/srep03594. During this operation, the culture was carried out using a dish coated in advance with a laminin 111 E8 fragment, in which domain I (Gly²⁵-Pro¹⁹⁶) of a human perlecan fragment (hereinafter also referred to as Pln-D1) is fused to the C-terminus of a human laminin α1-chain E8 fragment, (hereinafter also referred to as perlecan-modified LM111 E8, P-LM111 E8) [prepared in accordance with the method described in PCT/JP2020/029855, Japanese Patent Application No. 2020-132547 and Japanese Patent Application No. 2019-144899], dissolved in DPBS as a scaffolding molecule in a concentration of 0.5 µg/coating area cm².

### (2) Preparation of human iPS cell-derived neural crest stem (NCS) cells

The iPS cells cultured in (1) were differentiated into NCS cells based on the method described in Nature protocols, 2010, 5:688-701, or Cell reports, 2013, 3:1140-1152. The iPS cells were cultured for 5 days to 2 weeks in StemFit (registered trademark) AK02N (additive C(-)) culture medium containing noggin (manufactured by R&D systems, Inc., catalog No: 6057-NG-100/CF, 500 ng/mL) and/or SB431542 (manufactured by TOCRIS Bioscience, catalog No: 1614, 10 µM) thereby inducing to differentiation into NCS cells. No passaging is carried out during the differentiation induction of iPS cells to NCS cells, and hence P-LM111 E8 coated at the time of culturing iPS cells remains present as the scaffolding molecule.

### (3) Differentiation induction of NCS cells to SKPs

NCS cells prepared in (2) were cultured for 3 to 5 days in DMEM/F12 culture medium (manufactured by Life Technologies, catalog No: 10565-018) containing B-27 (trademark) supplement (manufactured by Life Technologies, catalog No: 17504-044, 2 mass%), EGF (manufactured by R&D systems, catalog No: 336-EG-200, 20 ng/mL), bFGF (manufactured by Wako, catalog No: 064-04541, 40 ng/mL), penicillin/streptomycin (manufactured by Life Technologies, catalog No: 15140-122, 50 U, 50 µg/mL), and CHIR99021 (manufactured by Cayman Chemical, catalog No: 13122, 3 µM) thereby inducing differentiation of NCS cells to SKPs. No passaging is carried out during the differentiation induction of NCS cells to SKPs, and hence P-LM111 E8 coated at the time of culturing iPS cells remains present as the scaffolding molecule. Subsequently, the cells differentiated into SKPs were subcultured without the P-LM111E8 scaffolding using a cultured cell detachment/dispersion solution (product name: Accutase, manufactured by BD Biosciences, catalog No: 561527) and then further cultured in D-MEM/F12 culture medium containing B-27 (trademark) supplement (2 mass%), EGF (20 ng/mL), bFGF (40 ng/mL), and penicillin/streptomycin (manufactured by Life Technologies, catalog No: 15140-122, 50 U, 50 µg/mL). The obtained SKPs were used for the following experiment as the dermal papilla cells.

### <Culture of pluripotent stem cell-derived dermal papilla cells on EMILIN-1>

### 1. Cell culture

Pluripotent stem cell-derived SKPs as dermal papilla cells as described above were seeded respectively on an uncoated dish (IWAKI: 3000-035) or on the same dish on which EM1-C in 0.5 µg/cm² was coated at 37°C and allowed to stand for 1 hour, and cultured for 3 days in an incubator of 37°C, 5%CO₂ using D-MEM/F12 culture medium (manufactured by Life Technologies, catalog No: 10565-018) containing B-27 supplement (manufactured by Life Technologies, catalog No: 17504-044, 2 mass%), EGF (manufactured by R&D Systems, catalog No:336-EG-200, 20 ng/mL), bFGF (manufactured by Wako, catalog No: 064-04541, 40 ng/mL), and penicillin/streptomycin (manufactured by Life Technologies, catalog No: 15140-122, 50 U, 50 µg/mL).

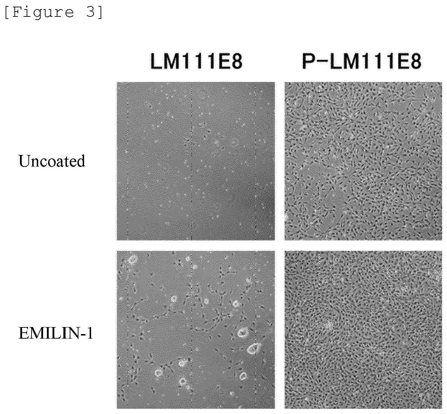

Figure 3 shows microscopic photographs of appearances of the obtained dermal papilla cells as cultured above. It appears that the pluripotent stem cell-derived dermal papilla cells also had excellent growth when cultured on EMILIN-1.

### 2. Gene expressions of pluripotent stem cell-derived dermal papilla cells

Gene expression state of the cultured pluripotent stem cell-derived dermal papilla cells was investigated by the quantitative PCR method. RNA was extracted from each of the samples using RNeasy Mini kit. Each total RNA concentration was measured, and reverse transcription was carried out using a given amount of the total RNA. For the reverse transcript, High capacity RNA-to-cDNA Kit was used. The gene expression by the quantitative PCR method was detected and quantified using 1 µL of the obtained cDNA sample and Taqman Probe by QuantStudio Realtime PCR system. The amplification conditions were denaturation reactions for 95 and 15 seconds in a 20-µL reaction system, 1 minute-annealing at 60°C, and an extension reaction. For each of the gene reaction levels, a value calculated by the ΔCt method was corrected by using an expression level of RPLP0, and the gene expression levels are each expressed as a relative value with the expression level in dermal papilla cells cultured on the collagen type I set to 1.0.

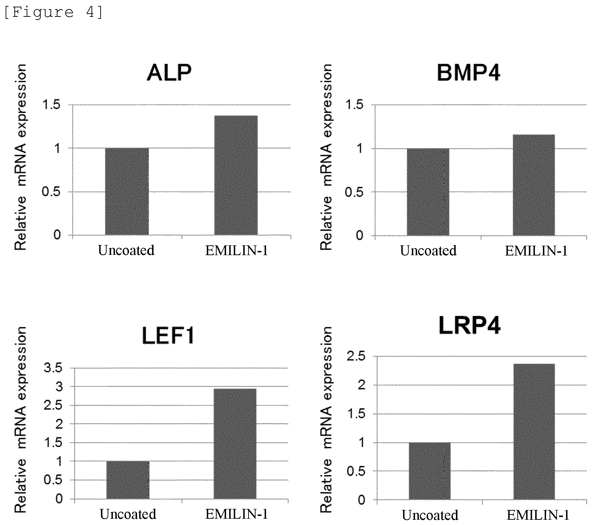

Figure 4 shows the results on the gene expression of pluripotent stem cell-derived dermal papilla cells investigated by the quantitative PCR method. It was confirmed that culture on EM1-C of the pluripotent stem cell-derived dermal papilla cells prepared using the P-LM111E8 fragment had an increased expression of ALP, which has been implicated in the ability to induce hair follicles. Further confirmed were increased expressions of BMP4 involved in hair follicle development during the fetal stage and LEF1 which suppresses the E-cadhelin expression at the time of developing hair follicles anlage. It was further confirmed that LRP4, which is a dermal papilla cell marker, had an increased expression.

From the above results, it is believed that EMILIN-1 has a potential to improve the ability of pluripotent stem cell-derived dermal papilla cells to induce hair follicles.

## Claims

1. A method for culturing dermal papilla cells, comprising culturing dermal papilla cells in the presence of at least one selected from the group consisting of EMILIN and a fragment thereof.

2. The method according to claim 1, wherein the culturing is carried out on a culture substrate comprising at least one selected from the group consisting of EMILIN and a fragment thereof, or carried out by adding to a culture medium at least one selected from the group consisting of EMILIN and a fragment thereof.

3. The method according to claim 1 or 2, wherein the EMILIN is EMILIN-1.

4. The method according to any one of claims 1 to 3, wherein the fragment of the EMILIN comprises gC₁q domain.

5. The method according to claim 4, wherein the fragment of the EMILIN is a C-terminal fragment of EMILIN-1.

6. A method for maintaining or facilitating an ability of dermal papilla cells to induce hair follicles, comprising culturing dermal papilla cells in the presence of at least one selected from the group consisting of EMILIN and a fragment thereof.

7. An agent for maintaining or facilitating an ability of dermal papilla cells to induce hair follicles, comprising as an active ingredient at least one selected from the group consisting of EMILIN and a fragment thereof.

8. Use of at least one selected from the group consisting of EMILIN and a fragment thereof for producing an agent for maintaining or facilitating an ability of dermal papilla cells to induce hair follicles.

9. Use of at least one selected from the group consisting of EMILIN and a fragment thereof for maintaining or facilitating an ability of dermal papilla cells to induce hair follicles.

10. The use according to claim 8 or 9, wherein the EMILIN is EMILIN-1.

11. The use according to any one of claims 8 to 10, wherein the fragment of the EMILIN comprises gC₁q domain.

12. The use according to claim 11, wherein the fragment of the EMILIN is a C-terminal fragment of EMILIN-1.

13. At least one selected from the group consisting of EMILIN and a fragment thereof for use in maintaining or facilitating an ability of dermal papilla cells to induce hair follicles.

14. The at least one selected from the group consisting of EMILIN and a fragment thereof according to claim 13, wherein the EMILIN is EMILIN-1.

15. The at least one selected from the group consisting of EMILIN and a fragment thereof according to claim 13 or 14, wherein the fragment of the EMILIN comprises gC₁q domain.

16. The at least one selected from the group consisting of EMILIN and a fragment thereof according to claim 15, wherein the fragment of the EMILIN is a C-terminal fragment of EMILIN-1.


SEQUENCE LISTING

<110>  KAO CORPORATIO
       OSAKA UNIVERSITY

<120>  Method for culturing dermal papilla cells

<130>  KS1676

<150>  2019-145099
<151>  2019-08-07

<160>  12

<170>  PatentIn version 3.5

<210>  1
<211>  1016
<212>  PRT
<213>  Homo sapiens

<400>  1

Met Ala Pro Arg Thr Leu Trp Ser Cys Tyr Leu Cys Cys Leu Leu Thr
1               5                   10                  15

Ala Ala Ala Gly Ala Ala Ser Tyr Pro Pro Arg Gly Phe Ser Leu Tyr
            20                  25                  30

Thr Gly Ser Ser Gly Ala Leu Ser Pro Gly Gly Pro Gln Ala Gln Ile
        35                  40                  45

Ala Pro Arg Pro Ala Ser Arg His Arg Asn Trp Cys Ala Tyr Val Val
    50                  55                  60

Thr Arg Thr Val Ser Cys Val Leu Glu Asp Gly Val Glu Thr Tyr Val
65                  70                  75                  80

Lys Tyr Gln Pro Cys Ala Trp Gly Gln Pro Gln Cys Pro Gln Ser Ile
                85                  90                  95

Met Tyr Arg Arg Phe Leu Arg Pro Arg Tyr Arg Val Ala Tyr Lys Thr
            100                 105                 110

Val Thr Asp Met Glu Trp Arg Cys Cys Gln Gly Tyr Gly Gly Asp Asp
        115                 120                 125

Cys Ala Glu Ser Pro Ala Pro Ala Leu Gly Pro Ala Ser Ser Thr Pro
    130                 135                 140

Arg Pro Leu Ala Arg Pro Ala Arg Pro Asn Leu Ser Gly Ser Ser Ala
145                 150                 155                 160

Gly Ser Pro Leu Ser Gly Leu Gly Gly Glu Gly Pro Gly Glu Ser Glu
                165                 170                 175

Lys Val Gln Gln Leu Glu Glu Gln Val Gln Ser Leu Thr Lys Glu Leu
            180                 185                 190

Gln Gly Leu Arg Gly Val Leu Gln Gly Leu Ser Gly Arg Leu Ala Glu
        195                 200                 205

Asp Val Gln Arg Ala Val Glu Thr Ala Phe Asn Gly Arg Gln Gln Pro
    210                 215                 220

Ala Asp Ala Ala Ala Arg Pro Gly Val His Glu Thr Leu Asn Glu Ile
225                 230                 235                 240

Gln His Gln Leu Gln Leu Leu Asp Thr Arg Val Ser Thr His Asp Gln
                245                 250                 255

Glu Leu Gly His Leu Asn Asn His His Gly Gly Ser Ser Ser Ser Gly
            260                 265                 270

Gly Ser Arg Ala Pro Ala Pro Ala Ser Ala Pro Pro Gly Pro Ser Glu
        275                 280                 285

Glu Leu Leu Arg Gln Leu Glu Gln Arg Leu Gln Glu Ser Cys Ser Val
    290                 295                 300

Cys Leu Ala Gly Leu Asp Gly Phe Arg Arg Gln Gln Gln Glu Asp Arg
305                 310                 315                 320

Glu Arg Leu Arg Ala Met Glu Lys Leu Leu Ala Ser Val Glu Glu Arg
                325                 330                 335

Gln Arg His Leu Ala Gly Leu Ala Val Gly Arg Arg Pro Pro Gln Glu
            340                 345                 350

Cys Cys Ser Pro Glu Leu Gly Arg Arg Leu Ala Glu Leu Glu Arg Arg
        355                 360                 365

Leu Asp Val Val Ala Gly Ser Val Thr Val Leu Ser Gly Arg Arg Gly
    370                 375                 380

Thr Glu Leu Gly Gly Ala Ala Gly Gln Gly Gly His Pro Pro Gly Tyr
385                 390                 395                 400

Thr Ser Leu Ala Ser Arg Leu Ser Arg Leu Glu Asp Arg Phe Asn Ser
                405                 410                 415

Thr Leu Gly Pro Ser Glu Glu Gln Glu Glu Ser Trp Pro Gly Ala Pro
            420                 425                 430

Gly Gly Leu Ser His Trp Leu Pro Ala Ala Arg Gly Arg Leu Glu Gln
        435                 440                 445

Leu Gly Gly Leu Leu Ala Asn Val Ser Gly Glu Leu Gly Gly Arg Leu
    450                 455                 460

Asp Leu Leu Glu Glu Gln Val Ala Gly Ala Met Gln Ala Cys Gly Gln
465                 470                 475                 480

Leu Cys Ser Gly Ala Pro Gly Glu Gln Asp Ser Gln Val Ser Glu Ile
                485                 490                 495

Leu Ser Ala Leu Glu Arg Arg Val Leu Asp Ser Glu Gly Gln Leu Arg
            500                 505                 510

Leu Val Gly Ser Gly Leu His Thr Val Glu Ala Ala Gly Glu Ala Arg
        515                 520                 525

Gln Ala Thr Leu Glu Gly Leu Gln Glu Val Val Gly Arg Leu Gln Asp
    530                 535                 540

Arg Val Asp Ala Gln Asp Glu Thr Ala Ala Glu Phe Thr Leu Arg Leu
545                 550                 555                 560

Asn Leu Thr Ala Ala Arg Leu Gly Gln Leu Glu Gly Leu Leu Gln Ala
                565                 570                 575

His Gly Asp Glu Gly Cys Gly Ala Cys Gly Gly Val Gln Glu Glu Leu
            580                 585                 590

Gly Arg Leu Arg Asp Gly Val Glu Arg Cys Ser Cys Pro Leu Leu Pro
        595                 600                 605

Pro Arg Gly Pro Gly Ala Gly Pro Gly Val Gly Gly Pro Ser Arg Gly
    610                 615                 620

Pro Leu Asp Gly Phe Ser Val Phe Gly Gly Ser Ser Gly Ser Ala Leu
625                 630                 635                 640

Gln Ala Leu Gln Gly Glu Leu Ser Glu Val Ile Leu Ser Phe Ser Ser
                645                 650                 655

Leu Asn Asp Ser Leu Asn Glu Leu Gln Thr Thr Val Glu Gly Gln Gly
            660                 665                 670

Ala Asp Leu Ala Asp Leu Gly Ala Thr Lys Asp Arg Ile Ile Ser Glu
        675                 680                 685

Ile Asn Arg Leu Gln Gln Glu Ala Thr Glu His Ala Thr Glu Ser Glu
    690                 695                 700

Glu Arg Phe Arg Gly Leu Glu Glu Gly Gln Ala Gln Ala Gly Gln Cys
705                 710                 715                 720

Pro Ser Leu Glu Gly Arg Leu Gly Arg Leu Glu Gly Val Cys Glu Arg
                725                 730                 735

Leu Asp Thr Val Ala Gly Gly Leu Gln Gly Leu Arg Glu Gly Leu Ser
            740                 745                 750

Arg His Val Ala Gly Leu Trp Ala Gly Leu Arg Glu Thr Asn Thr Thr
        755                 760                 765

Ser Gln Met Gln Ala Ala Leu Leu Glu Lys Leu Val Gly Gly Gln Ala
    770                 775                 780

Gly Leu Gly Arg Arg Leu Gly Ala Leu Asn Ser Ser Leu Gln Leu Leu
785                 790                 795                 800

Glu Asp Arg Leu His Gln Leu Ser Leu Lys Asp Leu Thr Gly Pro Ala
                805                 810                 815

Gly Glu Ala Gly Pro Pro Gly Pro Pro Gly Leu Gln Gly Pro Pro Gly
            820                 825                 830

Pro Ala Gly Pro Pro Gly Ser Pro Gly Lys Asp Gly Gln Glu Gly Pro
        835                 840                 845

Ile Gly Pro Pro Gly Pro Gln Gly Glu Gln Gly Val Glu Gly Ala Pro
    850                 855                 860

Ala Ala Pro Val Pro Gln Val Ala Phe Ser Ala Ala Leu Ser Leu Pro
865                 870                 875                 880

Arg Ser Glu Pro Gly Thr Val Pro Phe Asp Arg Val Leu Leu Asn Asp
                885                 890                 895

Gly Gly Tyr Tyr Asp Pro Glu Thr Gly Val Phe Thr Ala Pro Leu Ala
            900                 905                 910

Gly Arg Tyr Leu Leu Ser Ala Val Leu Thr Gly His Arg His Glu Lys

915                 920                 925

Val Glu Ala Val Leu Ser Arg Ser Asn Gln Gly Val Ala Arg Val Asp
    930                 935                 940

Ser Gly Gly Tyr Glu Pro Glu Gly Leu Glu Asn Lys Pro Val Ala Glu
945                 950                 955                 960

Ser Gln Pro Ser Pro Gly Thr Leu Gly Val Phe Ser Leu Ile Leu Pro
                965                 970                 975

Leu Gln Ala Gly Asp Thr Val Cys Val Asp Leu Val Met Gly Gln Leu
            980                 985                 990

Ala His Ser Glu Glu Pro Leu Thr  Ile Phe Ser Gly Ala  Leu Leu Tyr
        995                 1000                 1005

Gly Asp  Pro Glu Leu Glu His  Ala
    1010                 1015

<210>  2
<211>  34
<212>  DNA
<213>  Artificial Sequence

<220>
<223>  Synthetic oligonucleotide

<400>  2
atatatgcta gccactgtgg agcgccccgc catg                                34

<210>  3
<211>  20
<212>  DNA
<213>  Artificial Sequence

<220>
<223>  Synthetic oligonucleotide

<400>  3
tccctgcccc gcggctcctc                                                20

<210>  4
<211>  26
<212>  DNA
<213>  Artificial Sequence

<220>
<223>  Synthetic oligonucleotide

<400>  4
atgtcgtggc cggctcagtg acagtg                                         26

<210>  5
<211>  40
<212>  DNA
<213>  Artificial Sequence

<220>
<223>  Synthetic oligonucleotide

<400>  5
cctcctgctg cagcctgtta atctcagaaa tgatacggtc                              40


<210>  6
<211>  40
<212>  DNA
<213>  Artificial Sequence

<220>
<223>  Synthetic oligonucleotide

<400>  6
gaccgtatca tttctgagat taacaggctg cagcaggagg                              40


<210>  7
<211>  57
<212>  DNA
<213>  Artificial Sequence

<220>
<223>  Synthetic oligonucleotide

<400>  7
atatataagc ttctaatgat gatgatgatg atgcgcgtgt tcaagctctg ggtcccc           57


<210>  8
<211>  60
<212>  DNA
<213>  Artificial Sequence

<220>
<223>  Synthetic oligonucleotide

<400>  8
ccaggttcca ctggtgacca tcatcatcat catcatgagg agggacaagc acaggccggc        60


<210>  9
<211>  45
<212>  DNA
<213>  Artificial Sequence

<220>
<223>  Synthetic oligonucleotide

<400>  9
atatataagc ttctacgcgt gttcaagctc tgggtcccca tagag                        45


<210>  10
<211>  21
<212>  PRT

<213>  Mus musculus

<400>  10

Met Glu Thr Asp Thr Leu Leu Leu Trp Val Leu Leu Leu Trp Val Pro
1               5                   10                  15

Gly Ser Thr Gly Asp
            20

<210>  11
<211>  20
<212>  DNA
<213>  Artificial Sequence

<220>
<223>  Synthetic oligonucleotide

<400>  11
cggtaggcgt gtacggtggg                                                   20

<210>  12
<211>  41
<212>  DNA
<213>  Artificial Sequence

<220>
<223>  Synthetic oligonucleotide

<400>  12
atgatgatga tgatgatggt caccagtgga acctggaacc c                           41